# EUROPÄISCHE PATENTSCHRIFT

(11) **EP 1 950 560 B1**
(45) Veröffentlichungstag und Bekanntmachung des Hinweises auf die Patenterteilung: **17.11.2010**
(21) Anmeldenummer: 07001374.3
(22) Anmeldetag: 23.01.2007
(51) Int. Cl.: G01N 33/28

(54) **Verfahren zur quantitativen Analyse von Gasen in einem Transformatoröl**
Method for the quantitative analysis of gases in a transformer oil
Procédé destiné à l'analyse quantitative de gaz dans une huile de transformateur

(43) Veröffentlichungstag der Anmeldung: 30.07.2008
(73) Patentinhaber: EMH Energie-Messtechnik GmbH, 21438 Brackel (DE)
(72) Erfinder: Schröder, Karsten, 20146 Hamburg (DE)
(74) Vertreter: Seemann, Ralph

(56) Entgegenhaltungen:
- EP-A- 1 637 879
- DE-A1- 10 122 173
- DE-A1- 10 327 625
- US-A- 4 763 514
- US-B1- 6 289 716

## Beschreibung

Die Erfindung betrifft ein Verfahren und eine Einrichtung zur quantitativen Analyse von Gasen in einem Transformatoröl.

Transformatoren gehören zu den wichtigsten und kostenintensivsten Betriebsmitteln in der elektrischen Energieversorgung. Das Auftreten von Fehlern bei diesen Komponenten führt deshalb nicht nur zu Unterbrechungen in der elektrischen Energieversorgung, sondern auch zu wirtschaftlichen Schäden.

Ein Transformatorbetrieb soll eine kontinuierliche Energieversorgung möglichst über Jahre hinweg fehlerfrei sichern. Aus diesem Grund ist es erwünscht, dass auftretende Fehler, die eventuell Ausfälle der Transformatoren verursachen können, rechtzeitig erkannt werden, um geeignete Maßnahmen zur Fehlerbeseitigung einzuleiten.

Eine kontinuierliche Erfassung des Isolationszustandes kann somit große Fehler verhindern, die Nutzungsdauer eines Transformators verlängern und die Instandhaltung unterstützen.

Als Isoliermittel wird in Leistungstransformatoren eine Kombination aus einem flüssigen und einem festen Isolierstoff eingesetzt. Fehler in der Isolation von flüssigkeitsgefüllten Transformatoren sind fast immer von der Entwicklung in Öl gelöster Gase sowie einer Erhöhung der Ölfeuchte begleitet. Die Menge und Zusammensetzung der Zersetzungsgase hängt sowohl von der Isolierflüssigkeit als auch von der Art des zugrundeliegenden Fehlers und der dadurch freigesetzten Energiemenge ab. Fehler, durch die eine große Energiemenge freigesetzt wird, verursachen große Gasvolumina in kurzer Zeit, während die Gasvolumina bei kleineren Energiemengen verhältnismäßig gering sind. Ein Grund für die Entstehung der Gase ist beispielsweise die Zersetzung fester und flüssiger Isolierstoffe, die durch Teilentladung und Kreisströme, örtliche Überhitzungen durch Kurzschluss, hohe Übergangswiderstände oder starke Wirbelströme sowie durch Lichtbogenentladungen und -überschläge hervorgerufen werden. Dabei erfolgt durch elektrische und/oder thermische Energiezufuhr eine Zerstörung der langkettigen Ölmoleküle, wodurch insbesondere Wasserstoff und leichte Kohlenwasserstoffverbindungen, wie z.B. Ethylen und Acetylen, entstehen. Bei der Zersetzung von Cellulose entstehen zusätzlich Kohlenmonoxid und Kohlendioxid. Je nach Menge der entstandenen Gase können diese in gelöster und/oder ungelöster Form auftreten. Bei entsprechend aufgespaltenen Molekülen entstehen so auch Wassermoleküle, die zu einer unerwünschten Ölfeuchte führen.

Aus EP 1 637 879 A1 sind ein Verfahren und eine Vorrichtung zur quantitativen Analyse von Gasen in einem Transformatoröl bekannt. Dabei wird ein Gas vom Transformatoröl getrennt und in eine Messkammer eingebracht. Die Trennung des Gases vom Transformatoröl erfolgt durch ein Trennelement, das gasdurchlässig ist und Öl zurückhält, insbesondere handelt es sich dabei um eine Membran.

In DE 101 22 173 A1 werden ein Verfahren und eine Vorrichtung zur Überwachung von gelösten Gasen in flüssigkeitsgefüllten Hochspannungsanlagen beschrieben. Hierfür ist ein geschlossenes Separiergefäß vorgesehen, das mit Flüssigkeit aus einem Flüssigkeitstank befüllt wird. Aus dem Separiergefäß wird mit Hilfe einer Pumpe Flüssigkeit abgepumpt. Hierbei wird ein Gleichgewichtszustand zwischen Flüssigkeit und Gasphase im Separiergefäß aufrecht erhalten, der nur bei Unterbrechen des Pumpvorgangs verlassen wird. Dann kann sich der Flüssigkeitsspiegel im Separiergefäß erhöhen, und es wird eine Gasanalyse oberhalb des Flüssigkeitsspiegels im Separiergefäß vorgenommen.

US 4 763 514 A betrifft eine Extraktionskammer für in Isolieröl gelöstes Gas, die im Unterdruckverfahren betrieben wird. Dazu ist das Volumen der Extraktionskammer mittels einer beweglichen Platte veränderlich. Zum Nehmen einer Gasprobe wird zunächst das Volumen der Extraktionskammer minimiert und die Extraktionskammer vollständig mit Isolieröl gefüllt. Nach dem Herstellen einer repräsentativen Ölprobe in der Extraktionskammer wird diese abgesperrt und ihr Volumen unter Ausbildung eines Unterdrucks vergrößert, wodurch im Isolieröl gelöstes Gas ausgast und einen Raum in der Extraktionskammer oberhalb des Isolieröls ausfüllt. Anschließend wird das Öl aus der Extraktionskammer von oben zerstäubt erneut in die unter Unterdruck stehende Extraktionskammer eingeleitet, wodurch weiteres Gas ausgast. Anschließend erfolgt eine Einleitung des Gases in eine Messkammer.

Es ist Aufgabe der vorliegenden Erfindung, ein Verfahren und eine Einrichtung anzugeben, mit denen eine effiziente, zuverlässige und einfache quantitative Analyse von Gasen in einem Transformatoröl durchgeführt wird.

Die Aufgabe wird durch ein Verfahren zur quantitativen Analyse von Gasen in einem Transformatoröl gelöst, das die folgenden Schritte umfasst:
- Befüllen eines Trenngefäßes mit Transformatoröl, so dass ein Gasraum, der mit Umgebungsatmosphäre gefüllt ist, oberhalb des Transformatoröls im Trenngefäß verbleibt,
- Umwälzen des Transformatoröls in dem Trenngefäß mittels einer Pumpe,
- Befüllen einer Messkammer mit Gas aus dem Gasraum oberhalb des Transformatoröls,
- Messen der Konzentration wenigstens einer Gaskomponente in der Messkammer mit Hilfe einer, insbesondere in der Messkammer angeordneten, Sensorvorrichtung.

Als Vorteil dieses Verfahrens erweist es sich, dass das aus dem Transformatoröl gewonnene Gas einem darüber befindlichen Gasraum zur Messung der Konzentration von Gaskomponenten direkt entnommen wird. Für diese Art von Verfahren ist es nicht zwingend erforderlich, dass das Gas vor der Messung zunächst ein Trennelement durchströmt, welches das Gas hindurchtreten lässt, das Transformatoröl hingegen zurückhält.

In einer bevorzugten Ausführungsform des Verfahrens wird das Transformatoröl, insbesondere über eine Kapillare, in den Gasraum geleitet. Kapillare im Sinne der Erfindung bedeutet eine schmale Röhre, die gewährleistet, dass das Transformatoröl beim Eintritt in den Gasraum über die Kapillare eine vergleichsweise große Oberfläche zum Gasraum bildet, vergleichbar einem Springbrunnen.

Eine weitere bevorzugte Ausführungsform des Verfahrens sieht vor, dass die Geschwindigkeit, mit der das Transformatoröl in den Gasraum geleitet wird, über die Pumpe gesteuert wird.

Durch eine Steuerung der Geschwindigkeit ist es möglich, das Transformatoröl so in das Trenngefäß einzuleiten, dass dabei eine möglichst große Öloberfläche entsteht, d.h. eine möglichst große Trennfläche zwischen Öl und Gasraum. Auf diese Weise wird die Diffusion von Gas aus dem Transformatoröl in den darüber befindlichen Gasraum befördert.

In einer bevorzugten Ausführungsform des Verfahrens wird zwischen dem Befüllen des Trenngefäßes mit Transformatoröl und dem Befüllen der Messkammer mit Gas aus dem Gasraum oberhalb des Transformatoröls eine Zeitspanne vorgegeben, die eine Diffusion von Gas aus dem Transformatoröl in den Gasraum erlaubt.

Dadurch, dass eine solche Zeitspanne vorgegeben ist, wird ein einheitlicher Messstandard geschaffen. Dabei macht man sich den Umstand zunutze, dass aus Transformatoröl, welches einen hohen Gasanteil enthält, im gleichen Zeitraum mehr Gas diffundiert als aus einem Transformatoröl, in dem vergleichsweise wenig Gas gelöst ist. Die Gaskonzentrationen im Gasraum oberhalb des Transformatoröls nach einer einheitlich vorgegebenen Zeitspanne sind daher repräsentativ für die Menge an Gasen, die im Transformatoröl gelöst sind.

Die Zeitspanne wird mit Vorteil so gewählt, dass sie kleiner oder gleich der Zeitspanne ist, in der sich durch die Diffusion ein Gleichgewichtszustand zwischen der Konzentration der mindestens einen Gaskomponente im Gasraum und in dem Transformatoröl einstellt. Denn ein solches Verfahren sollte vorzugsweise zeitsparend arbeiten, und das Vorgeben einer Zeitspanne, die über den Zeitraum hinausgeht, der zum Erreichen eines Gleichgewichtszustands erforderlich ist, ist daher überflüssig. Von Bedeutung ist in diesem Zusammenhang, dass für das Verfahren auch eine Zeitspanne ausreicht, die kleiner ist als der Zeitraum, der zur Herstellung eines Gleichgewichtszustands zwischen der Konzentration der mindestens einen Gaskomponente im Gasraum und im Transformatoröls erforderlich ist. So ist es beispielsweise durch eine entsprechende Kalibrierung der für das Verfahren eingesetzten Sensorvorrichtung möglich, nach einer vorgegebenen Zeitspanne, die unterhalb des Zeitraums liegt, der zur Herstellung des Gleichgewichtszustands nötig ist, Rückschlüsse auf die Konzentration einzelner Gaskomponenten im Transformatoröl zu ziehen.

In praktischen Versuchen hat sich eine Zeitspanne im Rahmen zwischen 15 und 25 Minuten, insbesondere ungefähr bei 20 Minuten, bewährt.

Durch das Verfahren werden mit Hilfe einer geeigneten Sensorvorrichtung bevorzugt Konzentrationen einer oder mehrerer Gaskomponenten gemessen, die aus der Gruppe von H₂(Wasserstoff), CO(Kohlenmonoxid), C₂H₄(Ethylen) und C₂H₂(Acetylen) stammen.

Die Feuchtigkeit im Transformatoröl lässt sich mit Hilfe eines Feuchtesensors messen.

Ein besonderer Vorteil des Verfahrens liegt darin, dass anschließend die Messkammer belüftet und das Transformatoröl aus dem Trenngefäß abgeführt werden.

Vorzugsweise erfolgt dabei vor dem Abführen des Transformatoröls aus dem Trenngefäß in einem weiteren Schritt ein Befüllen des Trenngefäßes mit Transformatoröl, so dass nur ein geringer Gasraum oberhalb des Transformatoröls im Trenngefäß verbleibt.

Durch die fast vollständige Befüllung des Trenngefäßes mit Transformatoröl wird der Gasraum oberhalb des Transformatoröls ausgetauscht, so dass ein neuer Messvorgang mit unverfälschten Messwerten durchgeführt werden kann.

Mit Vorteil werden die Verfahrensschritte zyklisch wiederholt, so dass eine Überwachung von Gasen in einem Transformatoröl über einen längeren Zeitraum gewährleistet wird.

Für die Ausführung des erfindungsgemäßen Verfahrens geeignet ist eine Einrichtung zur quantitativen Analyse von Gasen in einem Transformatoröl mit einer Kapillaren zur Einleitung von Transformatoröl in ein Trenngefäß, einer mit dem Trenngefäß verbundenen Messkammer und mit einer Sensorvorrichtung zur Messung der Konzentration wenigstens einer Gaskomponente, die sich dadurch auszeichnet, dass eine Pumpe vorgesehen ist, die das Transformatoröl in dem Trenngefäß umwälzt, wobei die Pumpe den Füllstand des Transformatoröls in dem Trenngefäß steuert, so dass beim Befüllen des Trenngefäßes mit Transformatoröl ein Gasraum, der mit Umgebungsatmosphäre gefüllt ist, oberhalb des Transformatoröls im Trenngefäß verbleibt.

Durch eine derartige Umwälzung wird die Diffusion von Gas aus Transformatoröl in einen darüber befindlichen Gasraum im Trenngefäß befördert.

Mit der Sensorvorrichtung wird die Konzentration einer oder mehrerer Gaskomponenten aus der Gruppe von H₂ (Wasserstoff), CO (Kohlenmonoxid), C₂H₄ (Ethylen) und C₂H₂ (Acetylen) gemessen.

Dabei ist es möglich, mehrere Sensoren für die Erfassung der Konzentration einer Gaskomponente vorzusehen. Durch diese Redundanz lässt sich die Zuverlässigkeit der Messung erhöhen.

Als Sensoren sind beispielsweise infrarotoptische Sensoren geeignet, insbesondere in Bezug auf die Gase CO (Kohlenmonoxid), C₂H₄ (Ethylen) und C₂H₂ (Acetylen).

Die Einrichtung kann einen Feuchtesensor zur Messung der Feuchtigkeit im Transformatoröl umfassen. Der Feuchtesensor ist beispielsweise am Boden des Trenngefäßes angeordnet.

Die Erfindung wird nachstehend ohne Beschränkung des allgemeinen Erfindungsgedankens anhand von Ausführungsbeispielen unter Bezugnahme auf die Zeichnungen beschrieben. Bezüglich aller im Text nicht näher erläuterten erfindungsgemäßen Einzelheiten wird ausdrücklich auf die Zeichnungen verwiesen. Es zeigen:
- Fig. 1: eine erfindungsgemäße Einrichtung zur quantitativen Analyse von Gasen in einem Transformatoröl in einer perspektivischen Ansicht von schräg oben, wobei seitliche Wände des Gehäuses abgedeckt sind, um einen Einblick in die Einrichtung zu gewähren,
- Fig. 2: die Einrichtung aus Fig. 1, wobei zusätzlich die vordere Wand des Gehäuses mit Display und Bedientafel für einen besseren Einblick entfernt ist,
- Fig.3: eine Explosionsdarstellung einer erfindungsgemäßen Einrichtung zur quantitativen Analyse von Gasen in einem Transformatoröl ohne Gehäuse, und
- Fig. 4: eine Tabelle mit einer Übersicht über den zeitlichen Ablauf eines erfindungsgemäßen Verfahrens zur quantitativen Analyse von Gasen in einem Transformatoröl.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

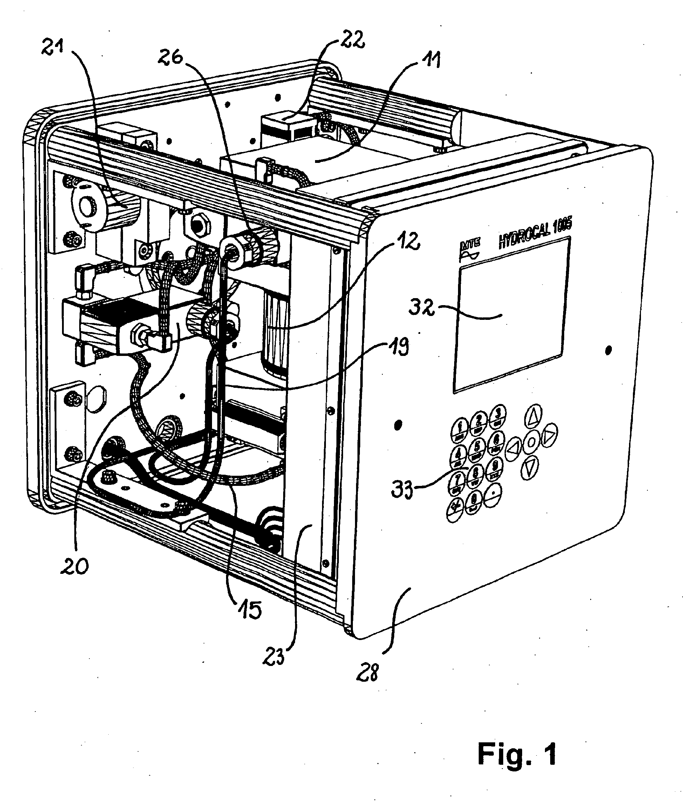

Fig. 1 zeigt eine erfindungsgemäße Einrichtung zur quantitativen Analyse von Gasen in einem Transformatoröl in perspektivischer Ansicht von schräg oben, wobei seitliche Wände des Gehäuses 28 abgedeckt sind, um einen Einblick in die Einrichtung zu gewähren.

Ein Trenngefäß 12 wird über ein Rohr 15 mit hier nicht sichtbarem Transformatoröl 13 befüllt. Die Zuleitung über das Rohr 15 wird über ein 3/2-Wege-Ventil 20 gesteuert. Im Trenngefäß 12 tritt im Transformatoröl 13 gelöstes Gas in einen darüber befindlichen Gasraum 27, der in der Figur 1 nicht dargestellt ist. Von dort gelangt es über eine Leitung 19 in eine Messkammer 11. Zwischen dem Trenngefäß 12 und der Messkammer 11 in der Leitung 19 befindet sich ein 2/2-Wege-Ventil 26, das entweder eine Verbindung zwischen Trenngefäß 12 und Messkammer 11 herstellt oder diese unterbricht. Zur Umwälzung des Transformatoröls 13 im Trenngefäß 12 ist eine Pumpe 21 vorgesehen. Für die Entleerung bzw. Belüftung der Messkammer 11 ist ein Kompressor 22 vorgesehen. In der Messkammer 11 befindet sich eine in der Fig. 1 nicht dargestellte Sensorvorrichtung 16, 17, 36, 37. Die durch die Sensorvorrichtung 16, 17, 36, 37 gemessenen Werte für die Konzentration wenigstens einer Gaskomponente in der Messkammer 11 werden an eine Systemelektronik 23 weitergegeben, dort verarbeitet und schließlich auf einem Display 32 angezeigt. Die Bedienung des Displays 32 erfolgt über eine Bedientafel 33. Das Display 32 und die Bedientafel 33 sind in eine vordere Wand des Gehäuses 28 integriert.

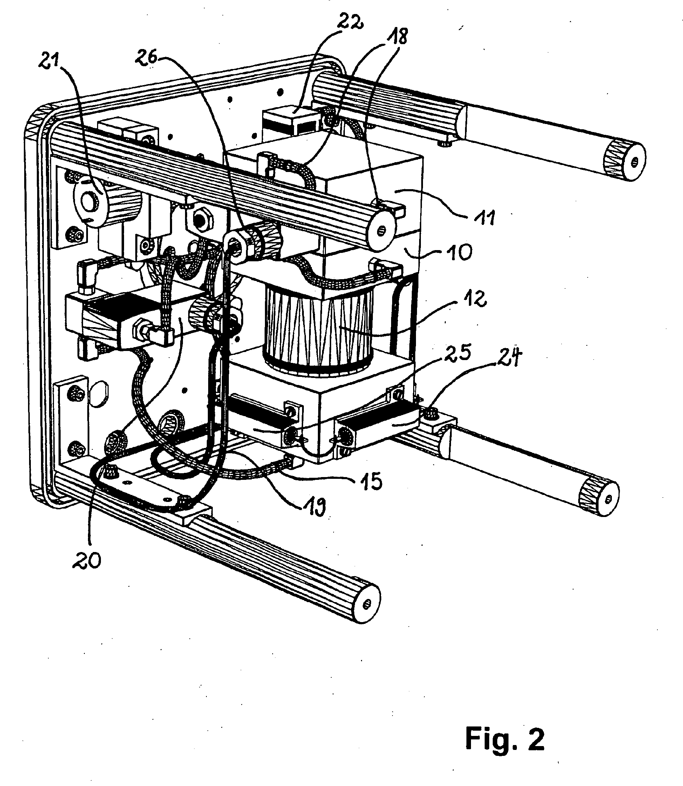

In Fig. 2 ist die Einrichtung aus Fig. 1 dargestellt, wobei zusätzlich die vordere Wand des Gehäuses 28 mit Display 32 und Bedientafel 33 für einen besseren Einblick in die Einrichtung entfernt wurde. So sind neben den bereits in der Fig. 1 vorgestellten Elementen der Einrichtung ein Trennelement 10 sowie ein Heizwiderstand 24 und ein Peltierelement 25 dargestellt. Das Trennelement 10 schirmt die Messkammer 11 mit der darin angeordneten und auch in Fig. 2 nicht sichtbaren Sensorvorrichtung 16, 17, 36, 37 vom Trenngefäß 12 ab. Gas aus dem in der Figur 2 nicht dargestellten Gasraum 27 oberhalb des Transformatoröls 13 im Trenngefäß 12 wird für eine Messung der Konzentration wenigstens einer Gaskomponente in die Messkammer 11 eingeleitet. Die von der Sensoreinrichtung 16, 17, 36, 37 gemessenen Werte werden über Kabel 18 als Signale an die Systemelektronik 23 weitergeleitet.

Der Heizwiderstand 24 dient dazu, Teile der Einrichtung aufzuheizen, und das Peltierelement 25 dient dazu, Teile der Einrichtung abzukühlen. Durch das Aufheizen und das Abkühlen können die Diffusionskoeffizienten der in ihrer Konzentration zu bestimmenden Gaskomponenten aus dem Transformatoröl 13 reproduzierbar konstant gehalten werden. Im Hinblick auf einen guten Austausch von Transformatoröl 13 hat es sich bewährt, mit Hilfe des Heizwiderstands 24 und des Peltierelements 25 einen Temperaturzyklus zu fahren.

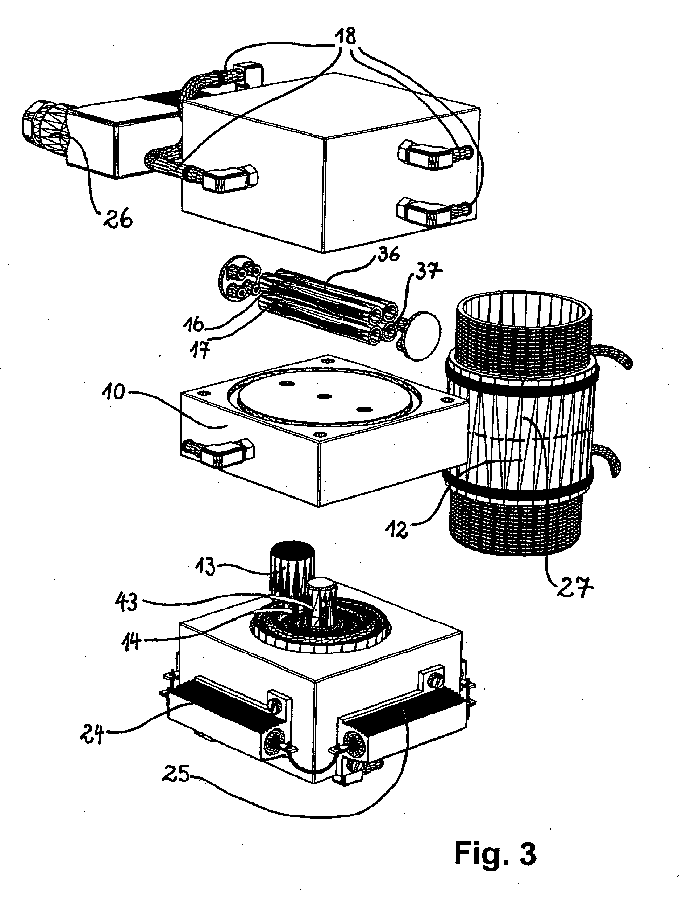

Die Fig. 3 stellt eine Explosionsdarstellung einer erfindungsgemäßen Einrichtung zur quantitativen Analyse von Gasen ohne Gehäuse dar. In der Fig. 3 ist die Sensorvorrichtung 16, 17, 36, 37 mit vier infrarotoptischen Sensoren zu sehen. Die Sensoren 16 und 17 können zum Beispiel identisch ausgebildete infrarotoptische Sensoren zur Messung von C₂H₄(Ethylen) und die Sensoren 36 und 37 können unterschiedlich ausgebildete infrarotoptische Sensoren zur Messung von CO(Kohlenmonoxid) und C₂H₂(Acetylen) sein. Ein Sensor zur Messung von H₂(Wasserstoff) ist in der Fig. 3 nicht dargestellt. Ein solcher Sensor ist vorzugsweise als elektrochemischer Sensor ausgebildet und in der Sensorvorrichtung 16, 17, 36, 37 separat von den infrarotoptischen Sensoren angeordnet. Des Weiteren ist ein Feuchtesensor 43 vorgesehen, der den Feuchtegehalt im Transformatoröl 13 misst. Der Feuchtesensor 43 in der Fig. 3 ist von einem Schutzgehäuse umgeben. Das Transformatoröl 13 strömt über eine Kapillare 14 in den Gasraum 27 im Trenngefäß 12. Dabei verlässt das Transformatoröl 13 die Kapillare 14 über eine nach oben gerichtete Öffnung in der Art eines Springbrunnens.

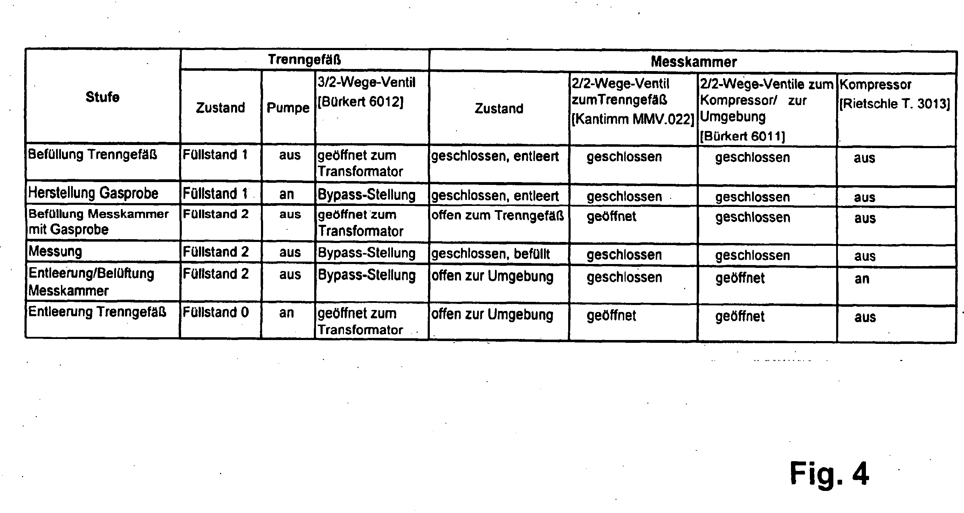

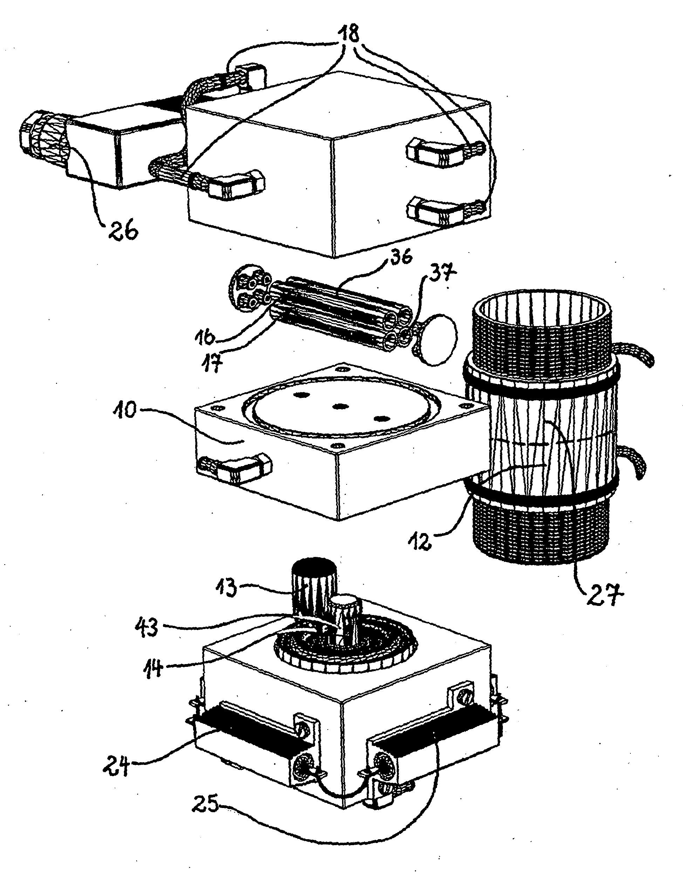

Die Fig. 4 zeigt eine Tabelle mit einer Übersicht über den zeitlichen Verlauf eines erfindungsgemäßen Verfahrens zur quantitative Analyse von Gasen in einem Transformatoröl 13. In der linken Spalte sind die einzelnen Verfahrensschritte oder Stufen angeführt. Hierzu werden für das Trenngefäß 12 der Zustand, d.h. der Füllstand, angegeben. "Füllstand 0" bedeutet ein im Wesentlichen leeres Trenngefäß 12, "Füllstand 1" bedeutet, dass das Trenngefäß 12 zum Teil gefüllt ist, so dass ein Gasraum 27 oberhalb des Transformatoröls 13 verbleibt. "Füllstand 2" bedeutet, dass sich soviel Transformatoröl 13 im Trenngefäß 12 befindet, dass nur ein geringer Gasraum 27 oberhalb des Transformatoröls 13 im Trenngefäß 12 vorliegt. Des Weiteren ist der Betriebszustand der Pumpe 21 angezeigt, die das Transformatoröl 13 über die Kapillare 14 in den Gasraum 27 pumpt. Die als Rohr 15 ausgebildete Verbindungsleitung zwischen dem Trenngefäß 12 und dem Transformator weist ein 3/2-Wege-Ventil vom Typ Bürkert 6012 auf, das dem Ventil 20 der Figuren 1 und 2 entspricht. Auf der rechten Seite der Tabelle sind Informationen in Bezug auf die Messkammer 11 aufgeführt. Die Messkammer 11 kann in verschiedenen Stufen entweder offen oder geschlossen, entweder entleert oder befüllt sein. Die Verbindungsleitung 19 zwischen der Messkammer 11 und dem Trenngefäß 12 weist ein 2/2-Wege-Ventil vom Typ Kantimm MMV.022 auf, das in den Figuren 1 und 2 dem Ventil 26 entspricht. Des Weiteren sind 2/2-Wege-Ventile vom Typ Bürkert 6011 zur Umgebung und zum Kompressor vom Typ Rietschle T. 3013, entsprechend dem Kompressor 22 in den Figuren 1 und 2, vorgesehen.

Im Folgenden wird ein Zyklus des Verfahrens beschrieben. Zunächst wird das Trenngefäß 12 bis zum Füllstand 1 mit Transformatoröl 13 befüllt. Dies kann beispielsweise dadurch erfolgen, dass das Transformatoröl 13 über die Pumpe 21 in das Trenngefäß 12 gedrückt wird. Das 3/2-Wege-Ventil zwischen Transformator und Trenngefäß 12 ist geöffnet. Die Messkammer 11 ist zu diesem Zeitpunkt noch geschlossen und enthält kein Gas, das gemessen wird. Geschlossen bedeutet in dieser Stufe, dass sämtliche Ventile zum Trenngefäß 12, zum Kompressor 22 und zur Umgebung geschlossen sind. Der Kompressor 22 ist nicht in Betrieb.

Zur Herstellung einer Gasprobe wird die Pumpe 21 in Betrieb genommen, die Transformatoröl 13 aus der Öffnung der Kapillaren 14 pumpt. Das 3/2-Wege-Ventil befindet sich in Bypass-Stellung. Der Zustand der Messkammer 11 ist in dieser Stufe unverändert.

In der folgenden Stufe erfolgt eine Befüllung der Messkammer 11 mit der Gasprobe. Hierfür wird die Gasprobe aus dem Trenngefäß 12 in die Messkammer 11 gedrückt. Ein Betrieb der Pumpe 21 ist in dieser Stufe nicht erforderlich. Das 3/2-Wege-Ventil ist zum Transformator geöffnet, um beim Befüllen der Messkammer 11 mit der Gasprobe nicht einer Sogwirkung entgegenarbeiten zu müssen. Das 2/2-Wege-Ventil zwischen Trenngefäß 12 und Messkammer 11 ist geöffnet, wohingegen die 2/2-Wege-Ventile zum Kompressor 22 und zur Umgebung geschlossen sind. Der Kompressor 22 bleibt ausgeschaltet.

In der nächsten Stufe erfolgt die Messung. Der Zustand des Trenngefäßes 12 ist bis auf die Stellung des 3/2-Wege-Ventils unverändert. Das 3/2-Wege-Ventil ist wieder in Bypass-Stellung. Die Messkammer 11 ist nun mit der Gasprobe befüllt, und sämtliche 2/2-Wege-Ventile sind geschlossen. In diesem Stadium erfolgt die Messung der Konzentration mindestens einer Gaskomponente in der Gasprobe.

Anschließend erfolgt die Entleerung bzw. Belüftung der Messkammer 11. Das Trenngefäß 12 befindet sich hierfür in unverändertem Zustand, und die Messkammer 11 wird über die 2/2-Wege-Ventile zum Kompressor 22 und zur Umgebung geöffnet, so dass ein Gasaustausch in der Messkammer 11 mit Hilfe des Kompressors 22 erfolgen kann.

In einer letzten Stufe erfolgt eine im Wesentlichen vollständige Entleerung des Trenngefäßes 12, d.h. das Transformatoröl 13 wird zum Transformator zurückgeleitet. Dies erfolgt mit Hilfe der Pumpe 21. Dabei ist das 3/2-Wege-Ventil in der Verbindungsleitung 15 zwischen Trenngefäß 12 und Transformator geöffnet. Sämtliche 2/2-Wege-Ventile der Messkammer sind in diesem Schritt geöffnet, der Kompressor 22 ist nicht in Betrieb.

### Bezugszeichenliste

- 10: Trennelement
- 11: Messkammer
- 12: Trenngefäß
- 13: Transformatoröl
- 14: Kapillare
- 15: Rohr
- 16: erster Sensor
- 17: zweiter Sensor
- 18: Kabel
- 19: Leitung
- 20: 3/2-Wege-Ventil
- 21: Pumpe
- 22: Kompressor
- 23: Systemelektronik
- 24: Heizwiderstand
- 25: Peltierelement
- 26: 2/2-Wege-Ventil
- 27: Gasraum
- 28: Gehäuse
- 32: Display
- 33: Bedientafel
- 36: dritter Sensor
- 37: vierter Sensor
- 43: Feuchtesensor

## Patentansprüche

1. Verfahren zur quantitativen Analyse von Gasen in einem Transformatoröl, umfassend die folgenden Schritte:
- Befüllen eines Trenngefäßes (12) mit Transformatoröl (13), so dass ein Gasraum (27), der mit Umgebungsatmosphäre gefüllt ist, oberhalb des Transformatoröls (13) im Trenngefäß (12) verbleibt,
- Umwälzen des Transformatoröls (13) in dem Trenngefäß (12) mittels einer Pumpe (21),
- Befüllen einer Messkammer (11) mit Gas aus dem Gasraum (27) oberhalb des Transformatoröls (13),
- Messen der Konzentration wenigstens einer Gaskomponente in der Messkammer (11) mit Hilfe einer, insbesondere in der Messkammer (11) angeordneten, Sensorvorrichtung (16, 17, 36, 37).

2. Verfahren nach Anspruch 1, **dadurch gekennzeichnet, dass** das Transformatoröl (13), insbesondere über eine Kapillare (14), in den Gasraum (27) geleitet wird.

3. Verfahren nach Anspruch 2, **dadurch gekennzeichnet, dass** die Geschwindigkeit, mit der das Transformatoröl (13) in den Gasraum (27) geleitet wird, über die Pumpe (21) gesteuert wird.

4. Verfahren nach einem der Ansprüche 1 bis 3, **dadurch gekennzeichnet, dass** zwischen dem Befüllen des Trenngefäßes (12) mit Transformatoröl (13) und dem Befüllen der Messkammer (11) mit Gas aus dem Gasraum (27) oberhalb des Transformatoröls (13) eine Zeitspanne vorgegeben wird, die eine Diffusion von Gas aus dem Transformatoröl (13) in den Gasraum (27) erlaubt.

5. Verfahren nach Anspruch 4, **dadurch gekennzeichnet, dass** die Zeitspanne kleiner oder gleich der Zeitspanne ist, in der sich durch die Diffusion ein Gleichgewichtszustand zwischen der Konzentration der mindestens einen Gaskomponente im Gasraum (27) und in dem Transformatoröl (13) einstellt.

6. Verfahren nach Anspruch 4 oder 5, **dadurch gekennzeichnet, dass** die Zeitspanne zwischen 15 und 25 Minuten, insbesondere ungefähr bei 20 Minuten, liegt.

7. Verfahren nach einem der Ansprüche 1 bis 6, **dadurch gekennzeichnet, dass** mit der Sensorvorrichtung (16, 17, 36, 37) die Konzentration einer oder mehrerer Gaskomponenten aus der Gruppe von H₂(Wasserstoff), CO(Kohlenmonoxid), C₂H₄(Ethylen) und C₂H₂(Acetylen) gemessen wird.

8. Verfahren nach einem der Ansprüche 1 bis 7, **dadurch gekennzeichnet, dass** die Feuchtigkeit im Transformatoröl (13) mit Hilfe eines Feuchtesensors (43) gemessen wird.

9. Verfahren nach einem der Ansprüche 1 bis 8, **dadurch gekennzeichnet, dass** in einem sich anschließenden Schritt ein Belüften der Messkammer (11) und ein Abführen des Transformatoröls (13) aus dem Trenngefäß (12) erfolgen.

10. Verfahren nach Anspruch 9, **dadurch gekennzeichnet, dass** vor dem Abführen des Transformatoröls (13) aus dem Trenngefäß (12) in einem weiteren Schritt ein Befüllen des Trenngefäßes (12) mit Transformatoröl (13) erfolgt, so dass nur ein geringer Gasraum (27) oberhalb des Transformatoröls (13) im Trenngefäß (12) verbleibt.

11. Verfahren zur Überwachung von Gasen in einem Transformatoröl, **dadurch gekennzeichnet, dass** ein Verfahren nach einem der Ansprüche 1 bis 10 zyklisch wiederholt wird.

## Claims

1. A method for the quantitative analysis of gases in a transformer oil, including the following steps:
- Filling a separation vessel (12) with transformer oil (13) so that a gas space (27), which is filled with atmospheric air, remains in the separation vessel (12) above the transformer oil (13),
- Circulating the transformer oil (13) in the separation vessel (12) by means of pump (21),
- Filling a measuring chamber (11) with gas from the gas space (27) above the transformer oil (13),
- Measuring the concentration of at least one gas component in the measuring chamber (11) with the aid of a sensor device (16, 17, 36, 37), particularly disposed in the measuring chamber (11).

2. A method as claimed in claim 1, **characterised in that** the transformer oil (13) is conducted into the gas space (27), particularly by means of a capillary (14).

3. A method as claimed in claim 2, **characterised in that** the speed with which the transformer oil (13) is conducted into the gas space (27) is controlled by means of the pump (21).

4. A method as claimed in one of claims 1 to 3, **characterised in that** between the filling of the separation vessel (12) with transformer oil (13) and the filling of the measuring chamber (11) with gas from the gas space (27) above the transformer oil (13) there is a predetermined period of time, which permits diffusion of gas out of the transformer oil (13) into the gas space (27).

5. A method as claimed in claim 4, **characterised in that** the period of time is equal to or less than the period of time in which an equilibrium state between the concentration of the at least one gas component in the gas space (27) and in the transformer oil (13) occurs as a result of the diffusion.

6. A method as claimed in claim 4 or 5, **characterised in that** the period of time is between 15 and 25 minutes, particularly about 20 minutes.

7. A method as claimed in one of claims 1 to 6, **characterised in that** the concentration of one or more gas components from the group H₂ (hydrogen), CO (carbon monoxide), C₂H₄ (ethylene) and C₂H₂ (acetylene) is measured with the sensor device (16, 17, 36, 37).

8. A method as claimed in one of claims 1 to 7, **characterised in that** the moisture in the transformer oil (13) measured with the aid of a moisture sensor (43).

9. A method as claimed in one of claims 1 to 8, **characterised in that** ventilation of the measuring chamber (11) and draining of the transformer oil (13) from the separation vessel (12) occur in a subsequent step.

10. A method as claimed in claim 9, **characterised in that** before the draining of the transformer oil (13) from the separation vessel (12) in a further step, the separation vessel (12) is filled with transformer oil (13) so that only a small gas space (27) remains above the transformer oil (13) in the separation vessel (12).

11. A method of monitoring gases in a transformer oil, **characterised in that** a method as claimed in one of claims 1 to 7 is repeated cyclically.

## Revendications

1. Procédé d'analyse quantitative de gaz dans une huile pour transformateur, comprenant les étapes suivantes :
- remplissage d'une cuve de séparation (12) avec une huile de transformateur (13) de manière à laisser un espace gazeux (27) au-dessus de l'huile de transformateur (13) dans la cuve de séparation (12), ledit espace gazeux (12) étant à pression atmosphérique,
- mise en circulation de l'huile de transformateur (13) dans la cuve de séparation (12) au moyen d'une pompe (21),
- remplissage d'une chambre de mesure (11) avec du gaz provenant de l'espace gazeux (27) situé au dessus de l'huile de transformateur (13),
- mesure de la concentration d'au moins un composant gazeux dans la chambre de mesure (11) à l'aide d'un dispositif de capteurs (16, 17, 36, 37), notamment placé dans la chambre de mesure (11).

2. Procédé selon la revendication 1, **caractérisé en ce que** l'huile de transformateur (13) est dirigée dans l'espace gazeux (27), notamment via un capillaire (14).

3. Procédé selon la revendication 2, **caractérisé en ce que** la vitesse à laquelle l'huile de transformateur (13) est dirigée dans l'espace gazeux (27) est commandée par la pompe (21).

4. Procédé selon l'une quelconque des revendications 1 à 3, **caractérisé en ce que**, entre le remplissage de la cuve de séparation (12) avec de l'huile de transformateur (13) et le remplissage de la chambre de mesure (11) avec du gaz provenant de l'espace gazeux (27) situé au-dessus de l'huile de transformateur (13), une période permettant une diffusion de gaz à partir de l'huile de transformateur (13) dans l'espace gazeux (27) est prédéfinie.

5. Procédé selon la revendication 4, **caractérisé en ce que** ladite période est inférieure ou égale à la période pendant laquelle s'établit, sous l'effet de la diffusion, un état d'équilibre entre la concentration d'au moins un composant gazeux dans l'espace gazeux (27) et dans l'huile de transformateur (13).

6. Procédé selon la revendication 4 ou 5, **caractérisé en ce que** ladite période est comprise entre 15 et 25 minutes, plus particulièrement est égale à environ 20 minutes.

7. Procédé selon l'une quelconque des revendications 1 à 6, **caractérisé en ce que** la concentration d'un ou de plusieurs composants du groupe comprenant le H₂ (hydrogène), le CO (monoxyde de carbone), le C₂H₄ (éthylène) et le C₂H₂ (acétylène) est mesurée avec le dispositif de capteurs (16, 17, 36, 37).

8. Procédé selon l'une quelconque des revendications 1 à 7, **caractérisé en ce que** l'humidité est mesurée dans l'huile de transformateur (13) à l'aide d'un capteur d'humidité (43).

9. Procédé selon l'une quelconque des revendications 1 à 8, **caractérisé en ce qu'**il est effectué une aération de la chambre de mesure (11) et une évacuation de l'huile de transformateur (13) hors de la cuve de séparation (12), lors d'une étape consécutive.

10. Procédé selon la revendication 9, **caractérisé en ce qu'**il est effectué, lors d'une étape supplémentaire située avant l'étape d'évacuation de l'huile de transformateur (13) hors de la cuve de séparation (12), un remplissage de la cuve de séparation (12) avec de l'huile de transformateur (13), de sorte qu'il ne reste qu'un faible espace gazeux (27) au-dessus de l'huile de transformateur (13) dans la cuve de séparation (12).

11. Procédé de surveillance de gaz dans une huile de transformateur, **caractérisé en ce qu'**un procédé selon l'une quelconque des revendications 1 à 10 est répété cycliquement.
